# EUROPÄISCHE PATENTSCHRIFT

(11) **EP 0 669 319 B1**
(45) Veröffentlichungstag und Bekanntmachung des Hinweises auf die Patenterteilung: **02.09.1998**
(21) Anmeldenummer: 95106324.7
(22) Anmeldetag: 13.06.1991
(51) Int. Cl.: C07D 209/48, C07D 401/04, C07D 401/12, A01N 37/32

(54) **O-Benzyl-Oximether und diese Verbindungen enthaltende Pflanzenschutzmittel**
O-Benzyloxime ethers and plant-protecting agents containing them
Ethers d'oximes O-benziliques et produits phytosanitaires les contenants

(30) Priorität: 27.06.1990 DE 4020384; 27.06.1990 DE 4020388
(43) Veröffentlichungstag der Anmeldung: 30.08.1995
(62) Teilanmeldung aus: 91109684.0
(73) Patentinhaber: BASF AKTIENGESELLSCHAFT, 67056 Ludwigshafen (DE)
(72) Erfinder: Brand, Siegbert, Dr., D-67346 Speyer (DE); Kardorff, Uwe, Dr., D-68159 Mannheim (DE); Kirstgen, Reinhard, Dr., D-67434 Neustadt (DE); Müller, Bernd, Dr., D-67227 Frankenthal (DE); Oberdorf, Klaus, Dr., D-69117 Heidelberg (DE); Sauter, Hubert, Dr., D-68167 Mannheim (DE); Lorenz, Gisela, Dr., D-67434 Hambach (DE); Ammermann, Eberhard, Dr., D-64646 Heppenheim (DE); Künast, Christoph, Dr., D-67166 Otterstadt (DE); Harreus, Albrecht, Dr., D-67063 Ludwigshafen (DE)

(56) Entgegenhaltungen:
- EP-A- 0 244 786
- EP-A- 0 253 213
- EP-A- 0 370 629
- EP-A- 0 414 153
- EP-A- 0 414 572
- WO-A-90/07493

## Beschreibung

Die vorliegende Erfindung betrifft neue O-Benzyl-Oximether und ein Verfahren zur Bekämpfung von Schädlingen, insbesondere von Pilzen, Insekten, Nematoden und Spinnmilben mit diesen Verbindungen.

Es ist bekannt, substituierte Phenylessigsäureoximderivate als Fungizide (EP 253 213) zu Verwenden. Ihre Wirkung ist jedoch unbefriedigend.

Desweiteren sind fungizide Wirkstoffe bekannt, in denen die dem Rest 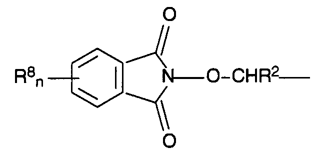 entsprechende Gruppe für einen Rest der Formel 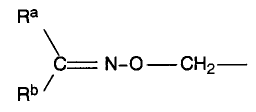 steht (EP-A 414 153, EP-A 370 692, WO-A 90/07,493).

Aus der Literatur (EP-A 414 572) sind außerdem Verbindungen mit wachstumsregulierender Wirkung bekannt, die sich von den erfindungsgemäßen Verbindungen strukturell dadurch unterscheiden, daß sie am N-(Oxyalkylen)-phthalimid keinen Phenylessigsäure-Rest tragen können.

Es wurde überraschend gefunden, daß O-Benzyl-Oximether der allgemeinen Formel I 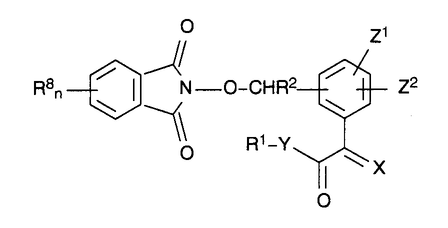 in der
- X: CH₂, CH-C₁-C₄-Alkyl, CH-C₁-C₄-Alkoxy, CH-C₁-C₄-Alkylthio oder N-C₁-C₄-Alkoxy bedeutet,
- Y: O, S oder NR⁵ bedeutet,
- R¹,R²,R⁵: H oder C₁-C₄-Alkyl bedeutet,
- Z¹, Z²: gleich oder verschieden sind und H, Halogen, Methyl, Methoxy oder Cyano bedeuten,
- n: die ganzen Zahlen 1 bis 4 bedeutet, und
- R⁸: gleich oder verschieden sind und H, Halogen, Cyano, Nitro; ggf. subst. C₁-C₄-Alkyl, C₁-C₄-Alkoxy, C₁-C₄-Halogenalkyl, C₁-C₄-Halogenalkoxy, Aryl, Aryloxy, Benzyloxy, Hetaryl oder Hetaryloxy bedeuten,
eine ausgezeichnete fungizide, insektizide, nematizide und akarizide Wirkung haben, die besser ist als die der bekannten Phenylessigsäurederivate.

Die fungizide Wirkung wird bevorzugt.

Die in der allgemeinen Formel I aufgeführten Reste können beispielsweise folgende Bedeutung haben:
- X: kann C₁-C₄-Alkyliden (z.B. Methyliden, Ethyliden, n- oder iso-Propyliden, n-, iso-, sec.- oder tert.-Butyliden), C₁-C₄-Alkoxymethyliden (z.B. Methoxy-, Ethoxy-, n- oder iso-Propoxy, n-, iso-, sec.- oder tert.-Butoxymethyliden), C₁-C₄-Alkylthiomethyliden (z.B. Methyl-, Ethyl-, n- oder iso-Propyl, n-, iso-, sec.- oder tert.-Butylthiomethyliden), C₁-C₄-Alkoxyimino (z.B. Methoxy-. Ethoxy-, n- oder iso-Propoxy-, n-, iso-, sec.- oder tert.-Butoxyimino),
- Y: kann 0, S, NR⁵,
- R¹,R²,R⁵: können H oder C₁-C₄-Alkyl (z.B. Methyl, Ethyl, n- oder iso-Propyl, n-, iso-, sec.- oder tert.-Butyl)
- Z¹, Z²: können H, Halogen (z.B. Fluor, Chlor, Brom, Jod), Methyl, Methoxy, Cyano
- n: kann 1, 2, 3 oder 4, und
- R⁸: kann z.B. H, Fluor, Chlor, Brom, Jod, Cyano, Nitro, Methyl, Ethyl, iso-Propyl, tert.-Butyl, Methoxy, Ethoxy, iso-Propoxy, tert.-Butoxy, Trifluormethyl, Chlormethyl, Dichlormethyl, Trichlormethyl, Trifluormethoxy, 1,1,2,2-Tetrafluormethoxy, Phenyl, Phenoxy, Benzyloxy, Pyrid-2-yl, bedeuten, wobei diese Reste wiederum durch Fluor, Chlor, Brom, Jod, Cyano, Methyl, Methoxy substituiert sein können.
Bevorzugt werden Verbindungen der Formel I, in der
- X: CH₂, CHCH₃, CHC₂H₅, CHOCH₃, CHOC₂H₅, CHSCH₃, CHSC₂H₅
- Y: O
- R¹: C₁-C₄-Alkyl,
- R²: H, Methyl
- Z¹, Z²: Wasserstoff, Fluor, Chlor, Brom, Jod, Methyl, Cyano oder Methoxy bedeuten.

Die neuen Verbindungen der allgemeinen Formel I können bei der Herstellung aufgrund der C=C- bzw. C=N-Doppelbindungen als E/Z-Isomerengemische anfallen. Diese können in der üblichen Weise, z.B. durch Kristallisation oder Chromatographie in die einzelnen Komponenten aufgetrennt werden. Sowohl die einzelnen isomeren Verbindungen als auch ihre Gemische werden von der Erfindung umfaßt und sind als Schädlingsbekämpfungsmittel brauchbar.

Die Herstellung der Verbindungen der allgemeinen Formel I gemäß Anspruch 1 erfolgt beispielsweise wie in Schema 1 beschrieben (Z¹, Z² gleich H).

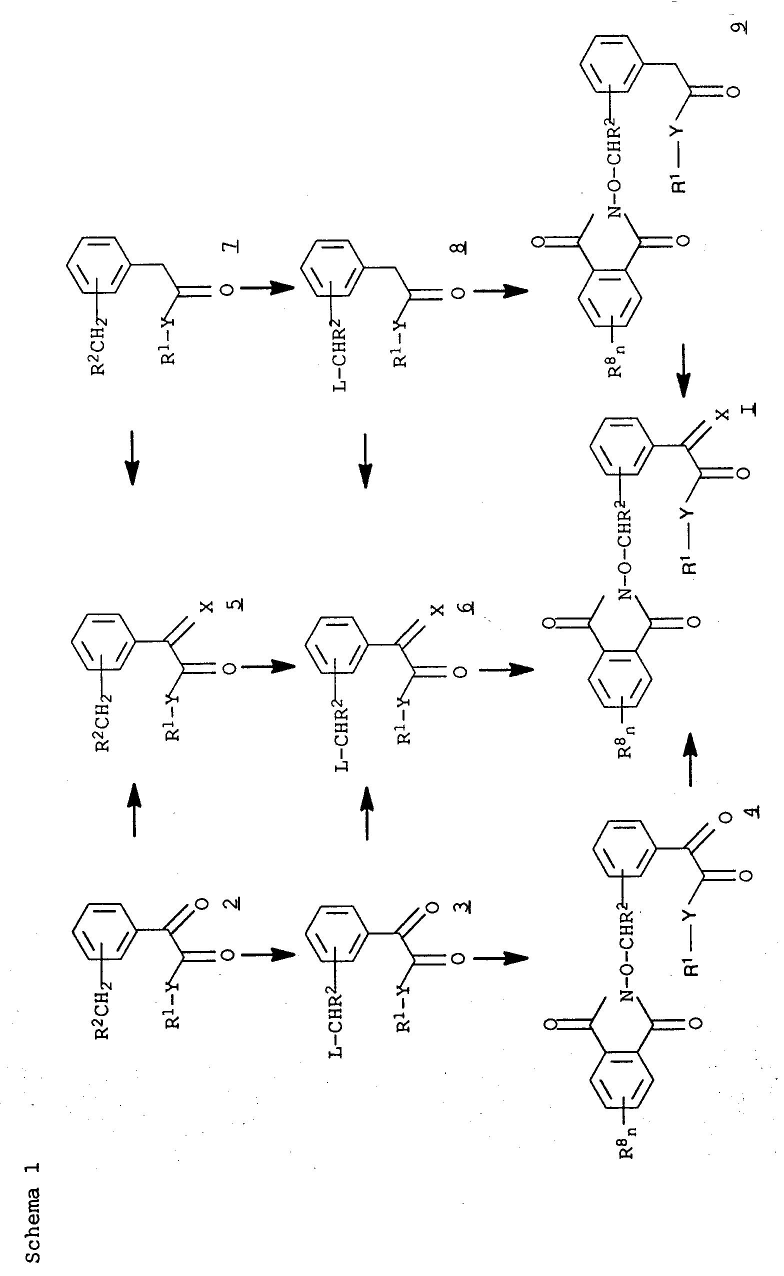

Die Verbindungen der allgemeinen Formel I, in denen X=CH₂, CH-Alkyl, CH-Alkoxy ist, lassen sich beispielsweise aus den Ketoestern 4 durch Wittig- oder Wittig-Horner-Reaktion herstellen (vgl. EP 348 766, DE 3 705 389, EP 178 826). Ebenso erhält man die analogen Verbindungen 5 aus den Ketoestern 2.

Alternativ kann auch so vorgegangen werden, daß man Verbindungen der Formel 7, bzw. 9 mit geeigneten Reagentien kondensiert, z.B. für X=CH₂ mit Formaldehyd (s. DE 3317356), für X=CH-Alkyl a) mit Aldehyden (vgl. D.M. Brown J. Chem. Soc. 1948, 2147) oder b) zuerst mit N,N-Dimethylformamiddimethylacetal, gefolgt von der Reaktion mit einem Grignardreagenz (analog zu C. Jutz Chem. Ber. 91, 1867 (1958)), für X=CH-O-Alkyl mit Ameisensäureester gefolgt von einer Alkylierung (s. EP 178826). Weitere Herstellvorschriften für die Verbindungen der Formel 5 und I mit X=CH-O-Alkyl sind beschrieben in EP 178 826.

Eine weitere Möglichkeit zur Herstellung der Verbindungen der Formel I mit X=CH-Alkyl und YR₂=COOAlk ist die Umsetzung von Ketenacetalen mit Phenylchlorcarbenen (s. N. Slougui, G. Rousseau, Synth. Commun. 12 (5) 401-7 (1982)).

Für Verbindungen der allgemeinen Formel I in der X=CH-S-Alkyl ist, kann die Herstellung nach den Methoden aus EP 244077 oder 310954 erfolgen.

Die Zwischenprodukte der Formeln 3, 6 und 8 lassen sich aus den Verbindungen, 2, 5 und 7 herstellen, indem man diese nach bekannten Methoden halogeniert, z.B. mit Chlor, Brom, N-Bromsuccinimid in einem inerten Lösungsmittel (z.B. CCl₄, Cyclohexan) unter Belichtung mit z.B. einer Hg-Dampflampe oder Radikalstartern, wie z.B. Dibenzoylperoxid, oder indem man über geeignete Zwischenverbindungen (L=Halogen, OH) die Reste L wie z.B. Mesylat, Tosylat, Acetat oder Triflat einführt.

Die Oximether der Formel I mit X=N-OAlkyl lassen sich aus 4a) durch Umsetzung mit O-Alkylhydroxylaminhydrochlorid oder b) mit Hydroxylaminhydrochlorid und nachfolgender Alkylierung mit einem Alkylierungsmittel (wie z.B. Alkyliodid, Dialkylsulfat etc.) herstellen (vgl. DE 3 623 921).

Ebenso kann analog zur Methode im EP 254 426 ein Phenylessigester der Formel 9 mit einer Base (wie z.B. NaOMe, NaH, K-tert. Butylat, etc.) in einem Lösungsmittel (wie z.B. Diethylether, Toluol, tert.-Butanol etc.) in sein Anion überführt und mit einem geeigneten Nitrosierungsmittel (wie z.B. Methylnitrit, Amylnitrit, tert.-Butylnitrit etc.) oximiert werden. Das resultierende Oximat wird mit einem Alkylierungsmittel (wie z.B. Alkyliodid, Dialkylsulfat) alkyliert.

Dieselben Verfahren sind entsprechend auch auf die Verbindungen der Formel 2 und 7 übertragbar, wobei die resultierenden Oximether 5 wie bekannt EP 254 426) über die Intermediate 6 (L=z.B. Halogen) in die Zielverbindungen I überführt werden können.

Üblicherweise ist bei den vorbeschriebenen Herstellungsverfahren der Rest Y-R¹ = Alkoxy.

Die Verbindungen mit YR¹=OH (11) lassen sich nach literaturbekannten Methoden (Organikum 16. Auflage, S. 415, 622) aus den Verbindungen der allgemeinen Formel I, in der YR¹ = COOAlkyl (10) ist, herstellen (s. Schema 2):

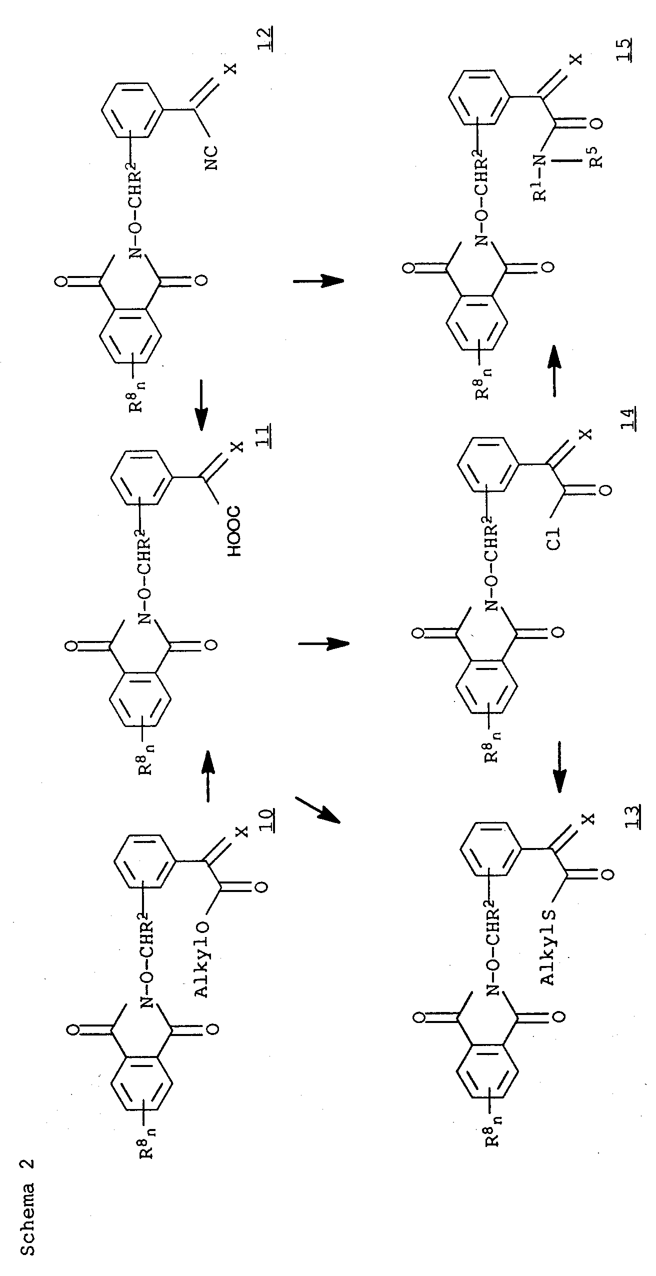

Alternativ dazu können die Nitrile 12 in bekannter Weise (vgl. Organikum 16. Auflage, S. 424f (1985)) in die Carbonsäuren 11 umgewandelt werden.

Aus den so erhaltenen Carbonsäuren 11 lassen sich in an sich bekannter Weise die Säurechloride 14 herstellen (vgl. Organikum 16. Auflage, S. 423f B. (1985)) . Die Umwandlung von 14 in die Amide 15 erfolgt analog zu Organikum 16. Auflage S. 412 (1985).

Die Thiolester 13 erhält man aus den Säurechloriden 14 (analog zu Houben-Weyl Bd. 8 S 464 ff (1952)).

Alternativ können die Thiolester 13 auch aus den Säuren 11 hergestellt werden (analog zu Houben-Weyl Bd E5 S. 855f (1985)).

Die Herstellung der Amide 15 mit R¹, R⁵=H kann auch nach literaturbekannten Verfahren aus den Nitrilen 12 erfolgen (vgl. dazu Synthesis 1980, 243).

Die Herstellung der Verbindungen der allgemeinen Formeln 2 und 7 mit ortho-Methylsubstitution am Aromaten (R²=H) ist bekannt.

(YR¹=OAlkyl; s. EP 178826, EP 260 832).

Einen weiteren Weg zur Herstellung der Verbindungen I (YR¹ = OAlkyl, X = CH-OAlkyl oder N-OAlkyl, L=Cl, Br, Tosylat, Mesylat) illustriert Schema 5:

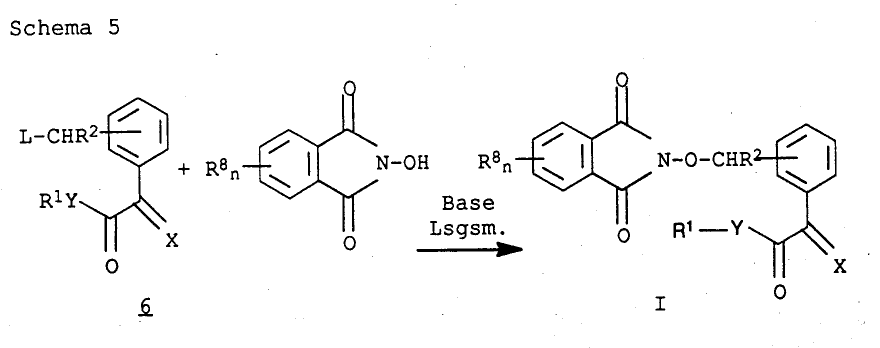

Diese Synthesesequenz erfolgt analog zu den in EP 244 786 beschriebenen Methoden.

So kann man ein N-Hydroxyphthalimid mit Halogeniden oder Sulfonsäureestern 6 in Gegenwart eines säurebindenden Mittels (z.B. Triethylamin, Kaliumcarbonat, etc.) in einem geeigneten Lösungsmittel (z.B. N-Methylpyrrolidon, DMF ect.) in die Imidether I überführen.

### Vorschrift 4:

### 3-Methoxy-2-[2'-(Phthalimidooxy)methyl]phenyl-acrylsäure-methylester (Nr. 1, Tab. I)

10 g (35 mmol) 3-Methoxy-2-(2'-Brommethyl)phenyl-acrylsäuremethylester, 5,7 g (35 mmol) Hydroxyphthalimid, 3,9 g (38,6 mmol) Triethylamin und 50 ml N-Methylpyrrolidon werden zusammengegeben und 2h bei 60°C gerührt. Dann gießt man auf Eiswasser, saugt den Rückstand ab, wäscht ihn mit Wasser und iso-Propanol und trocknet i. Vak.. Es verbleiben 9,0 g (70 % d.Th.) kristallines Produkt mit dem Schmelzpunkt 156-158°C.
¹H-NMR(CDCl₃): δ = 3,60 (s, 3H); 3,75 (s,3H); 5,12 (s,2H); 7,13 (dbr, 1H); 7,35 (m,2H); 7,62 (s,1H); 7,7-7,9(m, 5H).

### Beispiel 7

### 2-Methoximino-2-(2'-Phthalimidooxymethyl)phenylessigsäuremethylester (Nr. 1, Tab. II)

2,0 g (7 mmol) 2-Methoxyimino-2-(2'-Brommethyl)phenylessigsäuremethylester, 1,1 g /7 mmol) Hydroxyphthalimid, 0,8 g (7,7 mmol) Triethylamin werden in 10 ml N-Methylpyrrolidon gelöst und 2 h bei 70°C gerührt. Zur Aufarbeitung wird mit Eiswasser versetzt, die Kristalle abgesaugt, mit Wasser und Methyl-tert.-Butylether nachgewaschen und getrocknet.

Es verbleiben 1,5 g (58 % d. Th.) Kristalle mit dem Schmelzpunkt 152 - 155°C.
¹H-NMR(CDCl₃): δ = 3,83 (s, 3H); 3,98 (s,3H); 5,07 (s,2H); 7,15 (dbr, 1H); 7,45 (mc,2H); 7,60-7,85 (m,5H).

In entsprechender Weise lassen sich die in den Tabellen I-III aufgeführten Verbindungen herstellen.

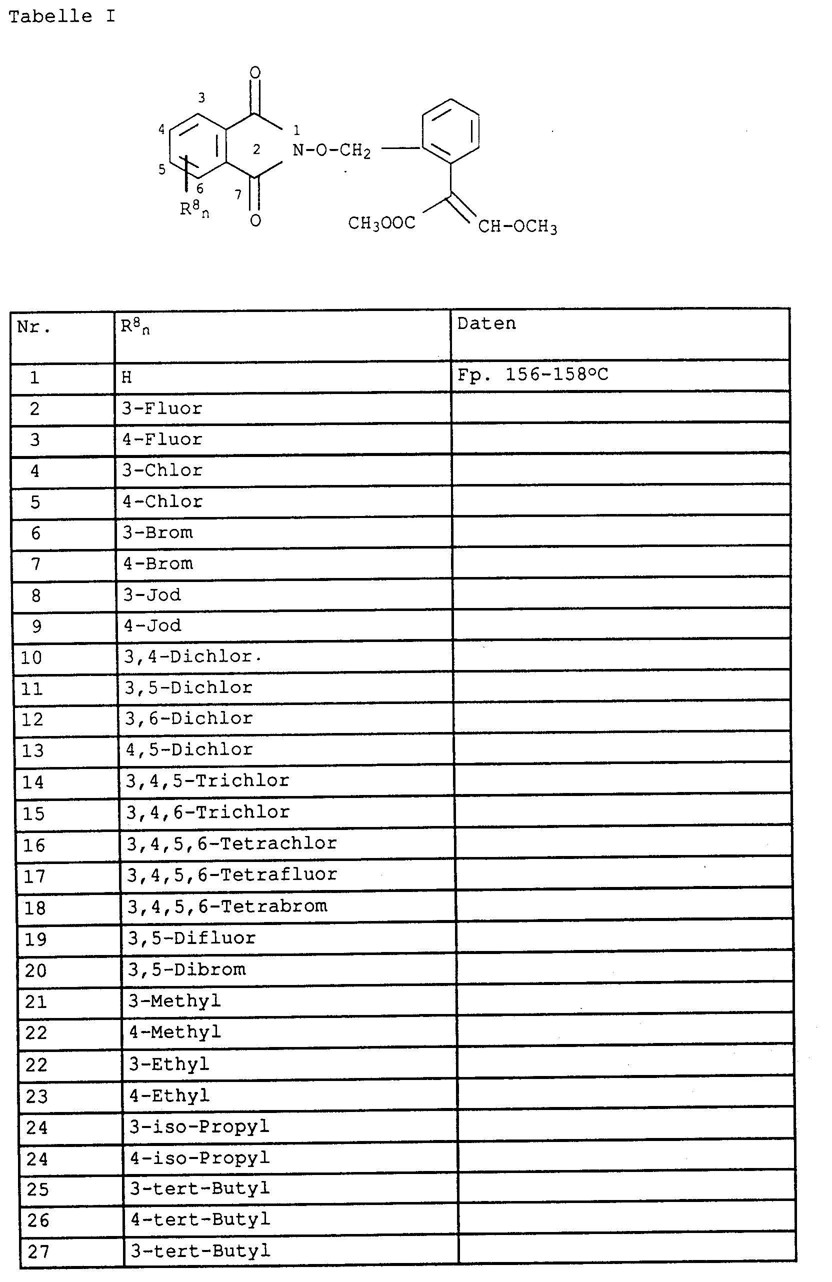

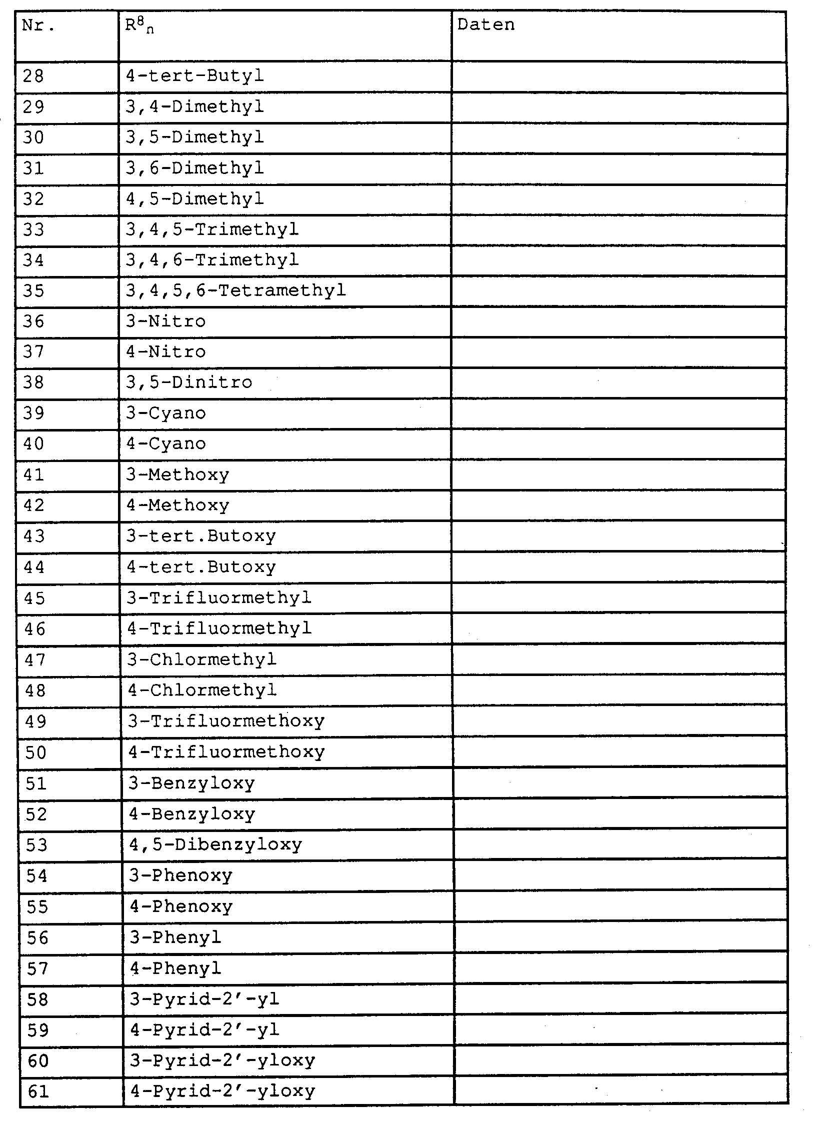

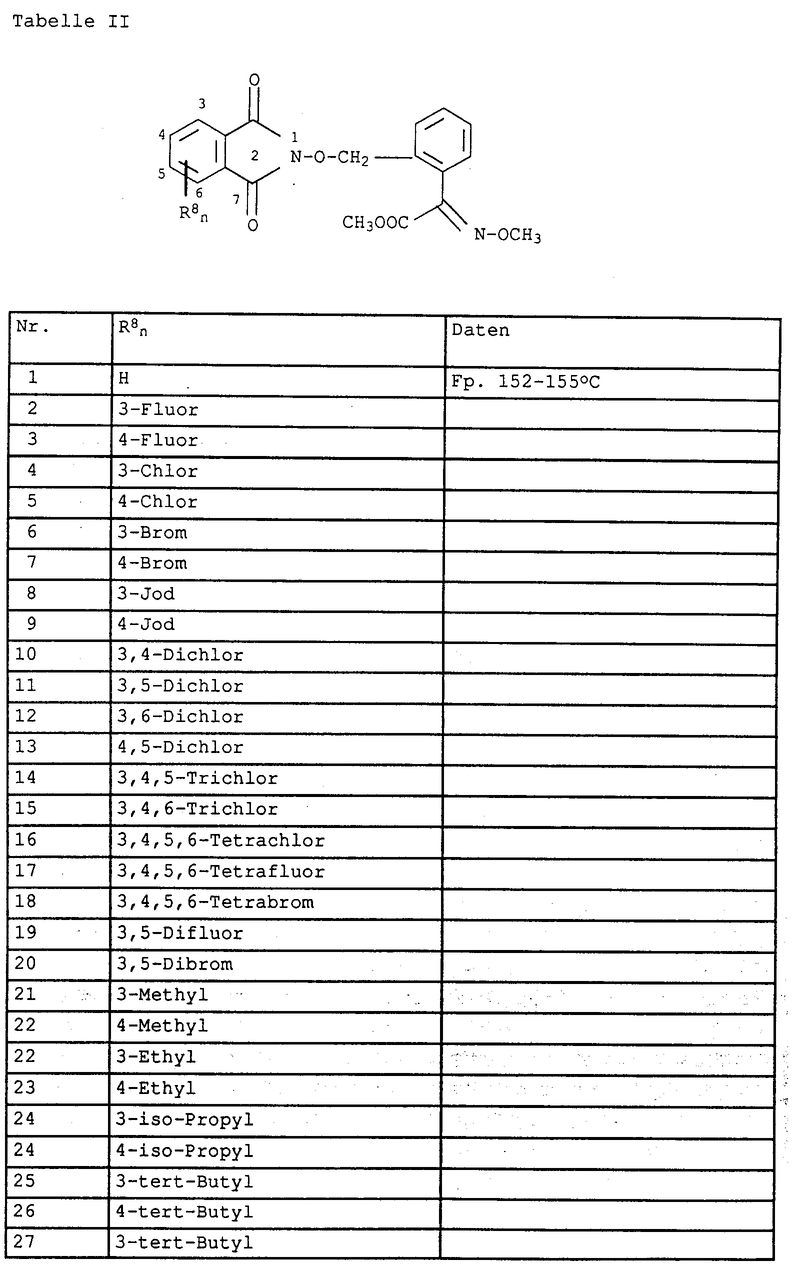

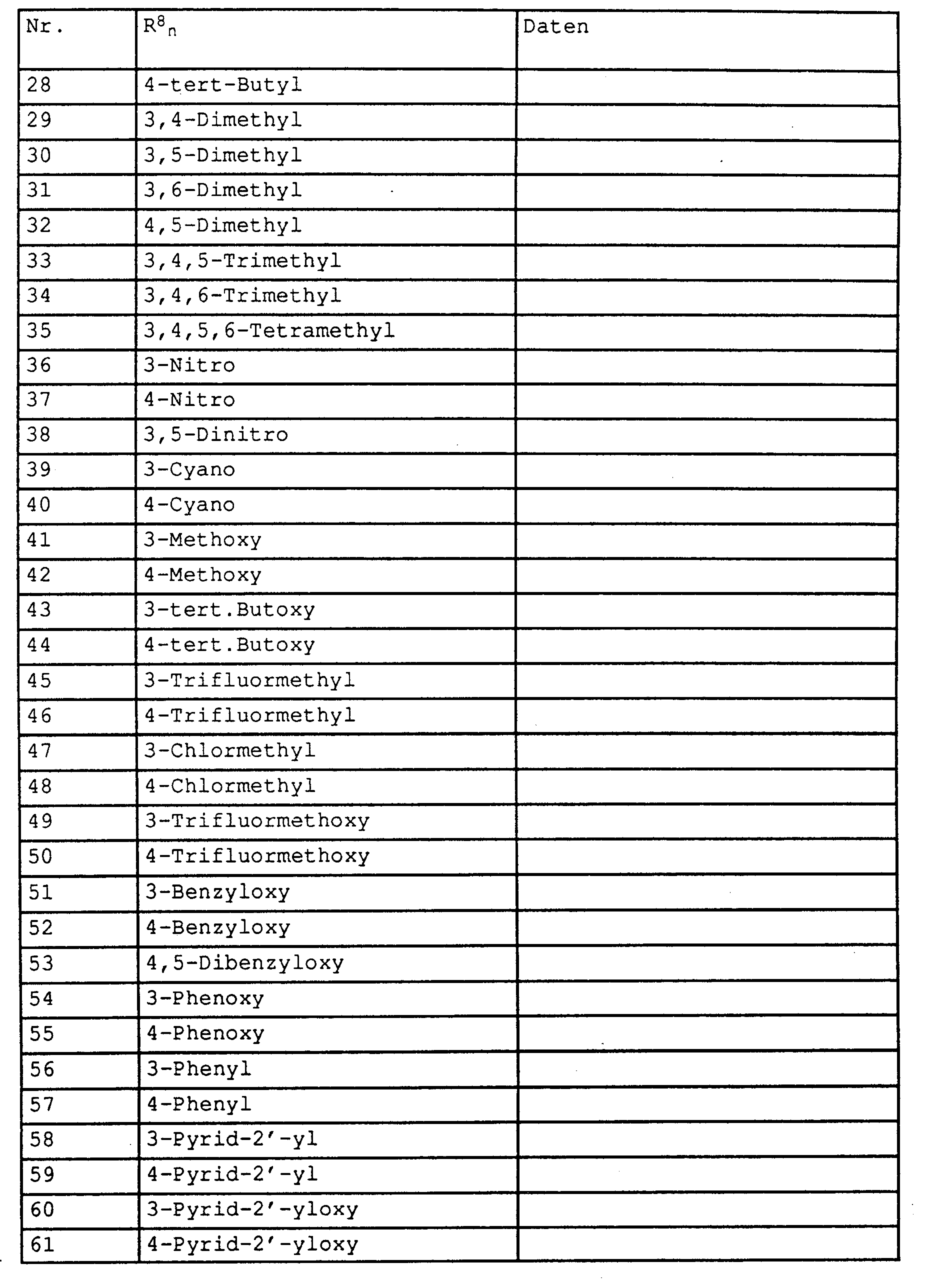

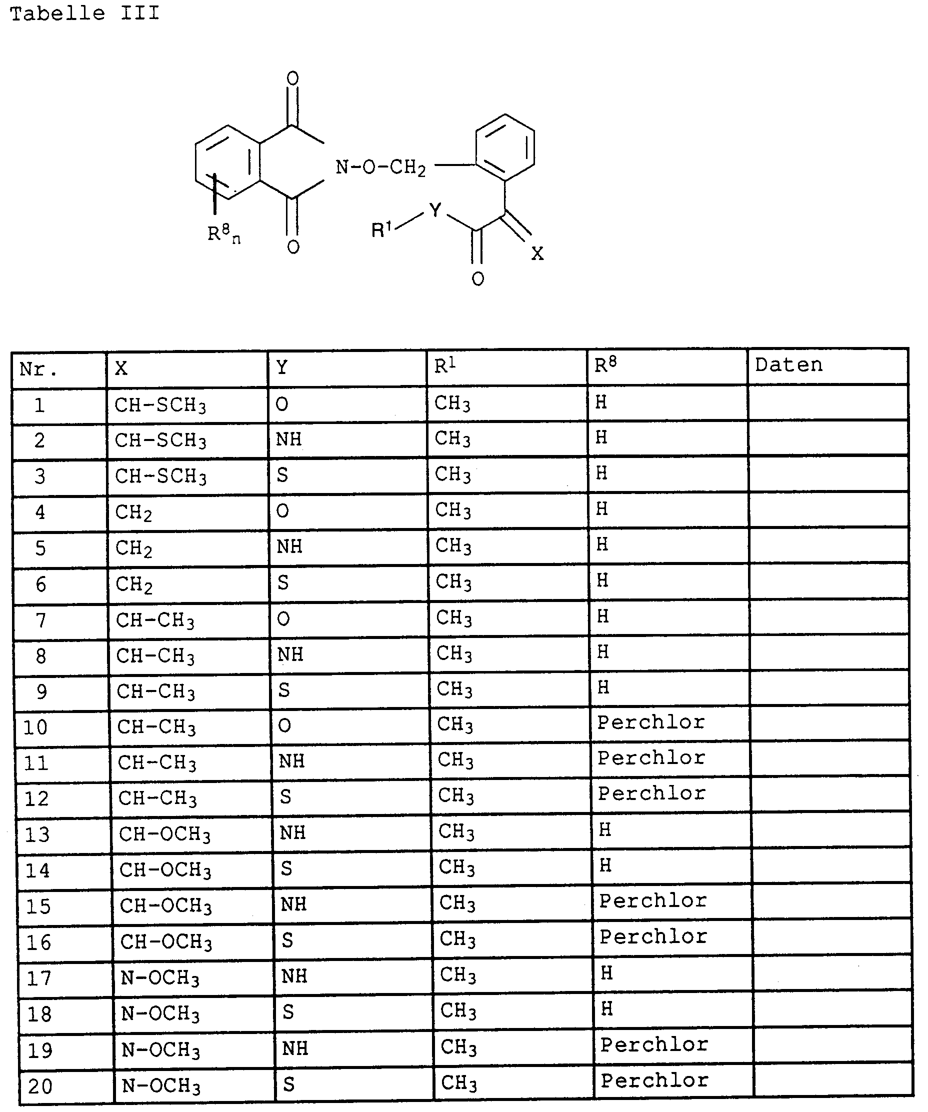

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:
Erysiphe graminis (echter Mehltau) in Getreide,
Erysiphe cichoracearum und Sphaerotheca fuliginea an Kürbisgewächsen, Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cerospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pflanzen mit den Wirstoffen besprüht oder bestäubt oder die Samen der Pflanzen mit den Wirkstoffen behandelt. Die Anwendung erfolgt vor oder nach der Infektion der Pflanzen oder Samen durch die Pilze. Es werden die Pilze oder die vor Pilzbefall zu schützenden Pflanzen, Saatgüter, Materialien oder der Erdboden mit einer fungizid wirksamen Menge des Wirkstoffs behandelt.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin-Sulfitablaugen und Methylcellulose.

Die fungiziden Mittel enthalten im allgemeinen zwischen 0,1 und 95, vorzugsweise zwischen 0,5 und 90 Gew.-% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz (Holzschutz) eingesetzt werden, z.B. gegen Paecilomyces variotii. Bei der Saatgutbehandlung werden im allgemeinen Wirkstoffmengen von 0,001 bis 50 g, vorzugsweise 0,01 bis 10 g, je Kilogramm Saatgut benötigt.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die neuen Verbindungen sind außerdem geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentailis, Cirphis unipuncta, Cydia pomonella, Dendrolimus pini, Diaphania nitidalis, Diatraea grandiosella, Earias insulana, Elasmopalpus lignosellus, Eupoecilia ambiguella, Evetria bouliana, Feltia subterranea, Galleria mellonella, Grapholita funebrana, Grapholita molesta, Heliothis armigera, Heliothis virescens, Heliothis zea, Hellula undalis, Hibernia defoliaria, Hyphantria cunea, Hyphantria cunea, Hyponomeuta malinellus, Keifferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flamea, Pectinophora gossypiella, Peridroma saucia, Phalera bucephala, Phthorimaea operculella, Phyllocnistis citrella, Pieris brassicae, Plathypena scarbra, Plutella xylostella, Pseudoplusia includens, Phyacionia frustrana, Scrobipalpula absoluta, Sitotroga cerelella, Sparganothis pilleriana, Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Onlema oryzae, Ortiorrhynchus sulcatus, Othiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chroysomya hominivorax, Chrysomya macellaria, Contarinia sorghicola, Cordylobia anthropophaga, Culex pipiens, Dacus cucurbitae, Dacus Oleae, Dasineura brassicae, Fannia canicularis, Gasterophilus intestinalis, Glossia morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweise Frankliniella fusc, Frankliniella occidentailis, Frankliniella tritici, Scirtothripis citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euchistus impictiventris, Leptoglussus phyllopus, Lygus lineolaris, Lygus pratensis, Nezara viridula, Piesma quadrata, Solubea insularis, Tyanta perditor.

Zur Klasse der Nematoden zählen beispielsweise Wurzelgallennematoden, z.B. Meloidogyne hapla, Meloidogyne incognita, Meloidogyne javanica, Zysten bildende Nematoden, z.B. Globodera rostochiensis, Heterodera avenae, Hetrodera glycinae, Heterodera schatii, Hetrodera triflolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynuchus dubius, Pratylenchus neglectus, Pratylenchus penetrans, Paratylenchus curvitatus, Partylenchus goodeyi.

Für die Anwendung zur Schädlingsbekämpfung können die Wirkstoffkonzentrationen in den anwendungsfertigen Zubereitungen in größeren Bereichen variiert werden.

Im allgemeinen liegen sie zwischen 0,0001 und 10 %, vorzugsweise zwischen 0,001 und 0,1 %.

Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Die Aufwandmenge an Wirkstoff für die Schädlingsbekämpfung beträgt unter Freilandbedingungen 0,01 bis 10, vorzugsweise 0,1 bis 1,0 kg/ha.

### Anwendungsbeispiel

Als Vergleichswirkstoff wurde 2-(Phenoxymethyl)-phenylglyoxylsäuremethylester-O-methyloxim (A) - bekannt aus EP 253 213 - benutzt.

### Wirksamkeit gegen Rebenperonospora

Blätter von Topfreben der Sorte "Müller Thurgau" wurden mit wäßriger Spritzbrühe, die 80 % Wirkstoff und 20 % Emulgiermittel in der Trockensubstanz enthielt, besprüht. Um die Wirkungsdauer der Wirkstoffe beurteilen zu können, wurden die Pflanzen nach dem Antrocknen des Spritzbelages 8 Tage im Gewächshaus aufgestellt. Erst dann wurden die Blätter mit einer Zoosporenaufschwemmung von Plasmopara viticola (Rebenperonospora) infiziert. Danach wurden die Reben zunächst für 48 Stunden in einer wasserdampfgesättigten Kammer bei 24°C und anschließend für 5 Tage in einem Gewächshaus bei Temperaturen zwischen 20 und 30°C aufgestellt. Nach dieser Zeit wurden die Pflanzen zur Beschleunigung des Sporangienträgerausbruchs abermals für 16 Stunden in der feuchten Kammer aufgestellt. Dann erfolgte die Beurteilung des Ausmaßes des Befalls der Blätter.

Das Ergebnis zeigt, daß aus Tabelle I Nr. 1, bei der Anwendung als 0,025 %ige (Gew.-%) Spritzbrühe eine bessere fungizide Wirkung zeigen (95 %) als der bekannte Vergleichswirkstoff A (50 %).

## Patentansprüche

1. O-Benzyl-Oximether der allgemeinen Formel I 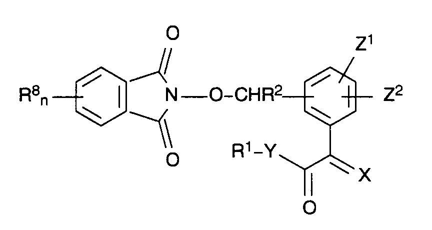 in der
X CH₂, CH-C₁-C₄-Alkyl, CH-C₁-C₄-Alkoxy, CH-C₁-C₄-Alkylthio oder N-C₁-C₄-Alkoxy bedeutet,
Y O, S oder NR⁵ bedeutet,
R¹,R²,R⁵ H oder C₁-C₄-Alkyl bedeutet,
Z¹, Z² gleich oder verschieden sind und H, Halogen, Methyl, Methoxy oder Cyano bedeuten,
n die ganzen Zahlen 1 bis 4 bedeutet, und
R⁸ gleich oder verschieden sind und H, Halogen, Cyano, Nitro; ggf. subst. C₁-C₄-Alkyl, C₁-C₄-Alkoxy, C₁-C₄-Halogenalkyl, C₁-C₄-Halogenalkoxy, Aryl, Aryloxy, Benzyloxy, Hetaryl oder Hetaryloxy bedeuten.

2. O-Benzyl-oximether der allgemeinen Formel II 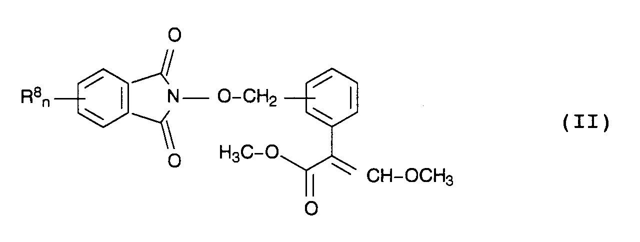 in der n und R⁸ die in Anspruch 1 gegebene Bedeutung haben.

3. O-Benzyl-oximether der allgemeinen Formel III 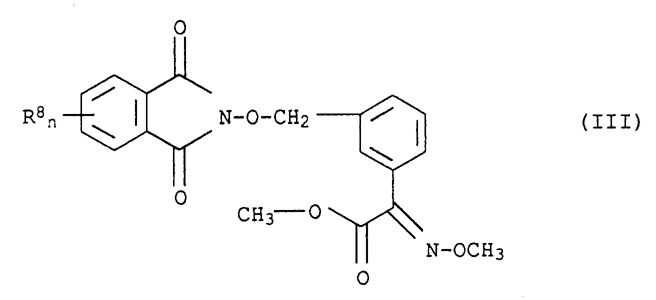 in der n und R⁸ die in Anspruch 1 gegebene Bedeutung haben.

4. Verfahren zur Herstellung von O-Benzyl-oximethern der Formel I gemäß Anspruch 1, dadurch gekennzeichnet, daß man ein Benzylderivat der Formel 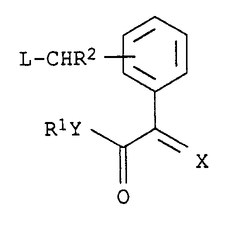 in der L eine Abgangsgruppe bedeutet, mit einem N-Hydroxyphthalimid der Formel 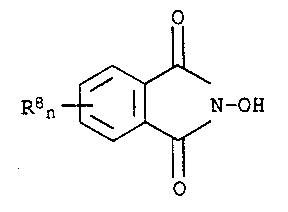 umsetzt.

5. Fungizid enthaltend einen inerten Trägerstoff und eine fungizid wirksame Menge eines O-Benzyl-oximethers der Formel I gemäß Anspruch 1.

6. Verfahren zur Bekämpfung von Pilzen, dadurch gekennzeichnet, daß man die Pilze oder die von Pilzbefall bedrohten Materialien, Pflanzen, Saatgut oder den Erdboden mit einer fungizid wirksamen Menge eines O-Benzyl-oximethers der Formel I gemäß Anspruch 1 behandelt.

7. Mittel zur Bekämpfung von Schädlingen aus der Klasse der Insekten, Spinnentiere und Nematoden, enthaltend einen inerten Trägerstoff und eine fungizid wirksame Menge eines O-Benzyloximethers der Formel I gemäß Anspruch 1.

8. Verfahren zur Bekämpfung von Schädlingen aus der Klasse der Insekten, Spinnentiere und Nematoden, dadurch gekennzeichnet, daß man die Schädlinge oder ihren Lebensraum mit einer wirksamen Menge eines O-Benzyloximethers der Formel I gemäß Anspruch 1 behandelt.

## Claims

1. O-benzyloxime ether of the formula I 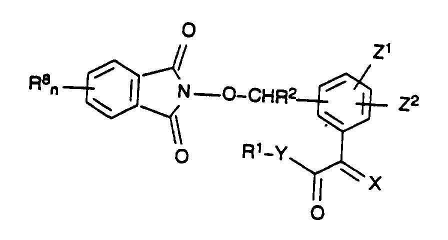 in which
X is CH₂, CH-C₁-C₄-alkyl, CH-C₁-C₄-alkoxy, CH-C₁-C₄-alkylthio or N-C₁-C₄-alkoxy,
Y is O, S or NR⁵,
R¹, R²₁ R ⁵ are each H or C₁-C₄-alkyl₁
Z¹, Z² are identical or different and are each H, halogen, methyl, methoxy or cyano,
n represents the integers from 1 to 4, and
R⁸ are identical or different and are each H, halogen, cyano, nitro; substituted or unsubstituted C₁-C₄-alkyl, C₁-C₄-alkoxy, C₁-C₄-haloalkyl, C₁-C₄-haloalkoxy, aryl, aryloxy, benzyloxy, hetaryl or hetaryloxy.

2. O-benzyloxime ether of the formula II 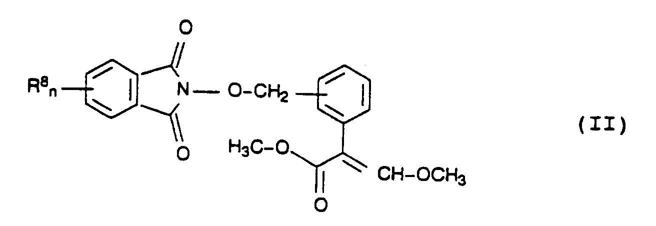 in which n and R⁸ are each as defined in Claim 1.

3. O-benzyloxime ether of the formula III 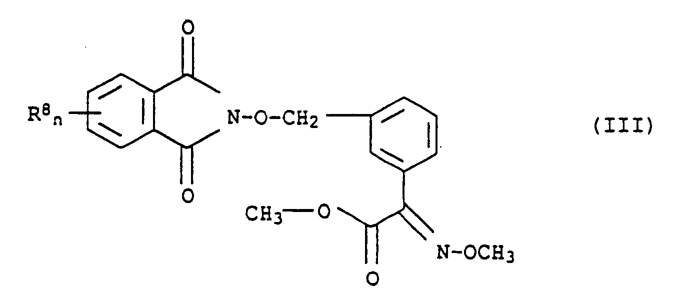 in which n and R⁸ are each as defined in Claim 1.

4. Process for preparing O-benzyloxime ethers of the formula I according to Claim 1, characterized in that a benzyl derivative of the formula 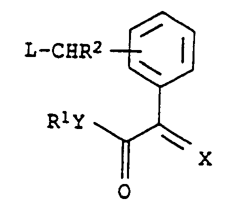 in which L is a leaving group, is reacted with an N-hydroxyphthalamide of the formula

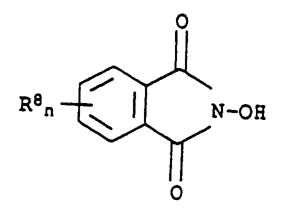

5. Fungicide, comprising an inert carrier and a fungicidally effective amount of an O-benzyloxime ether of the formula I according to Claim 1.

6. Method for controlling fungi, characterized in that the fungi or the materials, plants, seeds or the soil which are threatened by fungal attack are treated with a fungicidally effective amount of an O-benzyloxime ether of the formula I according to Claim 1.

7. Composition for controlling pests from the class of the insects, arachnids and nematodes, comprising an inert carrier and a fungicidally effective amount of an O-benzyloxime ether of formula I according to Claim 1.

8. Method for controlling pests from the class of the insects, arachnids and nematodes, characterized in that the pests or their habitat are treated with an effective amount of an O-benzyloxime ether of the formula I according to Claim 1.

## Revendications

1. Ethers d'O-benzyl-oximes de formule générale I 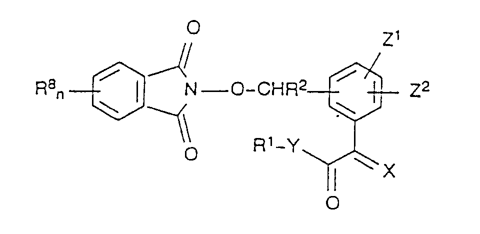 dans laquelle
X représente CH₂, CH-alkyle en C1-C4, CH-alcoxy en C1-C4, CH-alkylthio en C1-C4 ou N-alcoxy en C1-C4,
Y représente O, S ou NR⁵,
R¹, R², R⁵ représentent H ou des groupes alkyle en C1-C4,
Z¹ et Z², ayant des significations identiques ou différentes, représentent chacun H, un halogène, un groupe méthyle, méthoxy ou cyano,
n est un nombre entier allant de 1 à 4 et
les symboles R⁸, ayant des significations identiques ou différentes, représentent chacun H, un halogène, un groupe cyano, nitro ; un groupe alkyle en C1-C4, alcoxy en C1-C4, halogénoalkyle en C1-C4, halogénoalcoxy en C1-C4, aryle, aryloxy, benzyloxy, hétéroaryle ou hétéroaryloxy éventuellement substitué.

2. Ethers d'O-benzyl-oximes de formule générale II 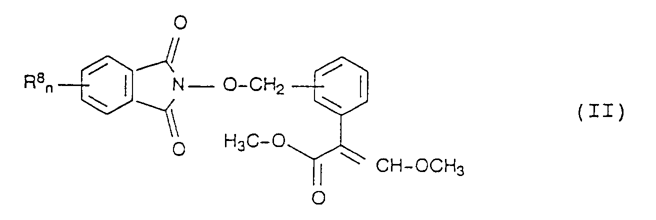 dans laquelle n et R⁸ ont les significations indiquées dans la revendication 1.

3. Ethers d'O-benzyl-oximes de formule générale III 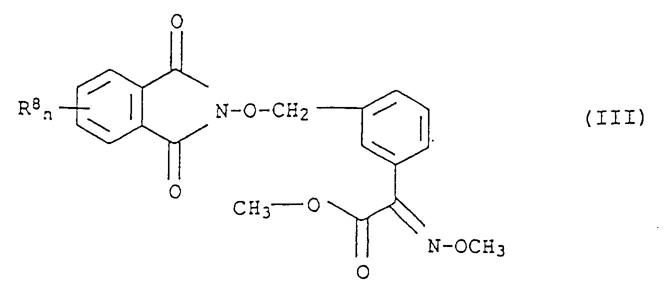 dans laquelle n et R⁸ ont les significations indiquées dans la revendication 1.

4. Procédé de préparation des éthers O-benzyl-oximes de formule I selon la revendication 1, caractérisé par le fait que l'on fait réagir un dérivé benzylique de formule 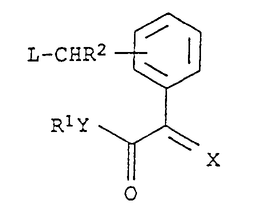 dans laquelle L représente un substituant éliminable, avec un N-hydroxyphtalimide de formule

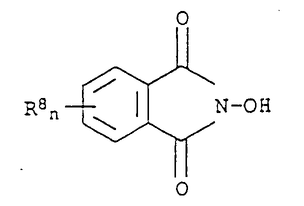

5. Produit fongicide contenant un véhicule inerte et une quantité fongicide efficace d'un éther d'O-benzyl-oxime de formule I selon la revendication 1.

6. Procédé pour combattre les mycètes, caractérisé par le fait que l'on traite les mycètes ou les matériaux, végétaux, semences ou sols menacés d'une attaque par les mycètes par une quantité fongicide efficace d'un éther d'O-benzyl-oxime de formule I selon la revendication 1.

7. Produit pour combattre les parasites de la classe des insectes, des arachnides et des nématodes, contenant un véhicule inerte et une quantité fongicide efficace d'un éther d'O-benzyl-oxime de formule I selon la revendication 1.

8. Procédé pour combattre les parasites de la classe des insectes, des arachnides et des nématodes, caractérisé par le fait que l'on traite les parasites ou leur habitat par une quantité efficace d'un éther d'O-benzyl-oxime de formule I selon la revendication 1.
